# EUROPEAN PATENT APPLICATION

(11) **EP 4 300 060 A1**
(43) Date of publication of application: **03.01.2024**
(21) Application number: 22182088.9
(22) Date of filing: 30.06.2022
(51) Int. Cl.: G01K 1/024, G01K 1/14, G01K 1/16, G01K 13/10, G01N 27/12, G01W 1/02, G01W 1/10, G01W 1/00

(54) **A GROUND MONITORING DEVICE**

(71) Applicant: Superhands OÜ, 10621 Tallinn (EE)
(72) Inventor: Pällo, Alvar, Viimsi (EE); Õis, Alari, Tallinn (EE); Tedremaa, Mihkel, Tallinn (EE); Metlitski, Stanislav, Tallinn (EE); Puust, Märt, Randvere (EE)
(74) Representative: Aaltonen, Janne Lari Antero

(57) **Abstract**

A ground monitoring device (100) for measuring ground surface data comprising a hollow cylindrical housing (110) having a first end (112) and a second end (114), wherein the hollow cylindrical housing comprises a power unit (120, 220, 320) in the first end (112), and an electronics module (130) in the second end (114). The electronics module (130) comprises at least one conductive element (131) comprising one or more first sensors, a radio transmitter (132, 232, 332), a power control real time clock (134), a computing element (135).

## Description

### TECHNICAL FIELD

The present disclosure belongs to autonomous wireless sensor solutions, more specifically to the systems for monitoring ground temperature, liquids content, salinity and other parameters.

### BACKGROUND

Existing sensor solutions for measuring ground data have physical installation limitations due to the size of the sensor, connectivity and power usage. Large dimensions of the sensors ruin homogeneity of the road. Therefore, road lifespan and measurement result accuracy are negatively affected. Additionally extra coordination and setup times are needed for installation. Cabled sensors require more cuts in the road surface, which damages the road surface and reduces the accuracy of the measurements. Currently known road data measuring sensors with extensive internal electronics have large dimensions and are prone to heat up due to internal and external heat dissipation. Measurement quality is thus worse when using large sensors. Due to extensive internal electronics the power consumption is challenging, the known sensors need to have external power source, large capacity battery or charging capabilities to last for years in active use, these all affects the size of the sensor, means higher costs or limited usage possibilities. Due to the large dimensions the currently known road data measurement sensors are not usable on the bridges because the sizes and in particular height is higher than the thickness of the usable surface layers of the bridges.

Therefore, in light of the foregoing discussion, there exists a need to overcome the aforementioned drawbacks associated with ground data monitoring.

### SUMMARY

The aim of the present disclosure is to provide an energy efficient and accurate ground data measurement device which would not damage the ground surface and its layers. The aim of the disclosure is achieved by a ground monitoring device for measuring ground surface data, comprising a hollow cylindrical housing having a first end and a second end, wherein the hollow cylindrical housing comprises a power unit in the first end, and an electronics module in the second end, wherein the electronics module comprises at least one conductive element comprising one or more first sensors, a radio transmitter, a second sensor, a power control real time clock, a computing element.

In another aspect, an embodiment of the present disclosure provides a computer program for measuring ground surface data comprising instructions which when the computer program is executed by a ground monitoring device comprising a computing element, cause the ground monitoring device to measure ground surface data.

The advantages of the present ground monitoring device are that it enables to reduce and optimize the power consumption, thus the ground monitoring device does not need charging, which makes the lifetime of the ground monitoring device longer than the lifetime of the prior art sensors. The ground monitoring device further enables to improve measurement accuracy and optimize the data transfer from the ground monitoring device to the external data communication units.

The present ground monitoring device enables to predict road conditions and slipperiness based on pavement temperature, present liquids on the surface. The information received from the sensor can be combined with, for example, weather station information, local geographical features, radar and satellite images, traffic and other data. The parameters measured by the ground monitoring device are input to the road weather forecasting model. The present ground monitoring device does not damage the road construction and can be used on all pavement types as well as on the bridges and other structures, e.g., viaducts) where construction and/or surface layers are thin. The ground monitoring device further enables to monitor agricultural ground surface data (e.g., temperature, liquids content, salinity).

Additional aspects, advantages, features and objects of the present disclosure would be made apparent from the drawings and the detailed description of the illustrative embodiments construed in conjunction with the appended claims that follow.

### BRIEF DESCRIPTION OF THE DRAWINGS

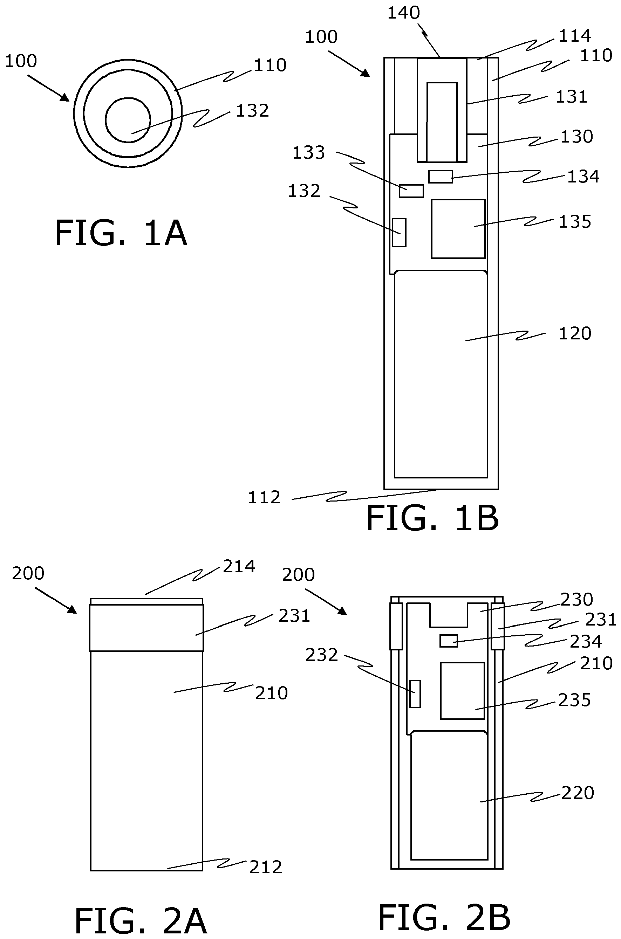

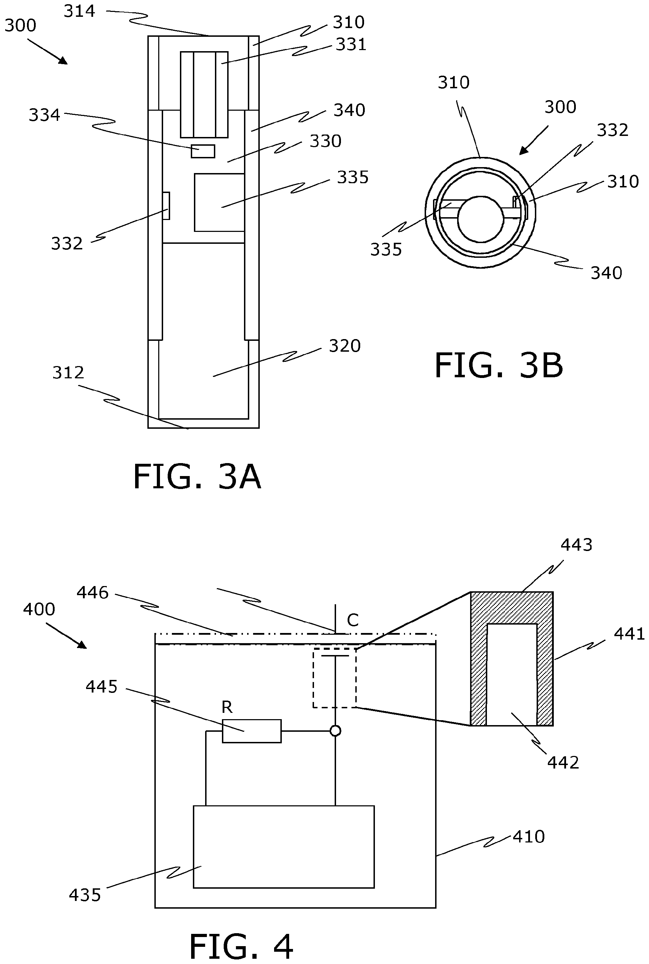

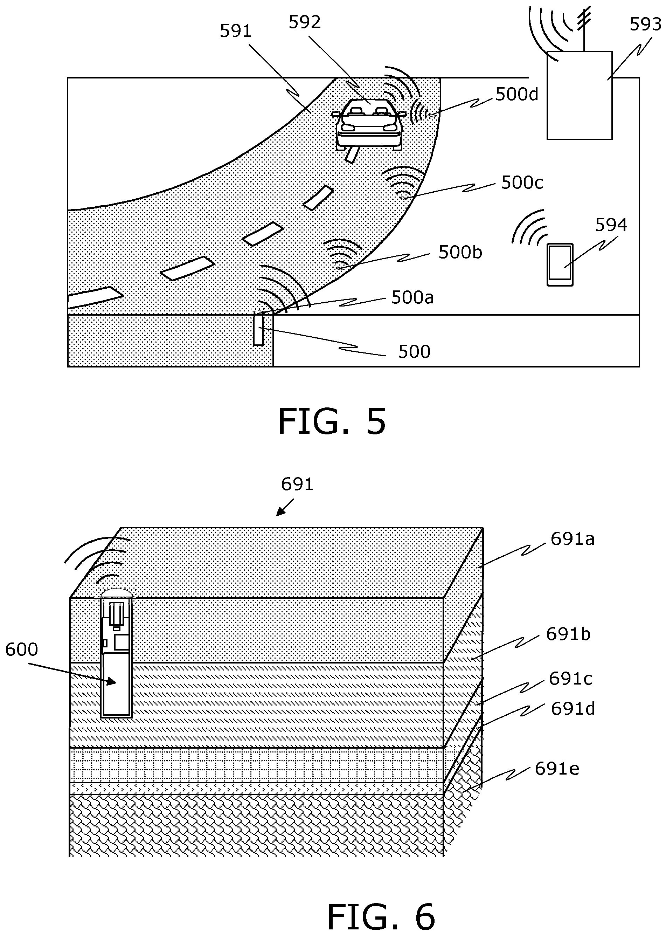

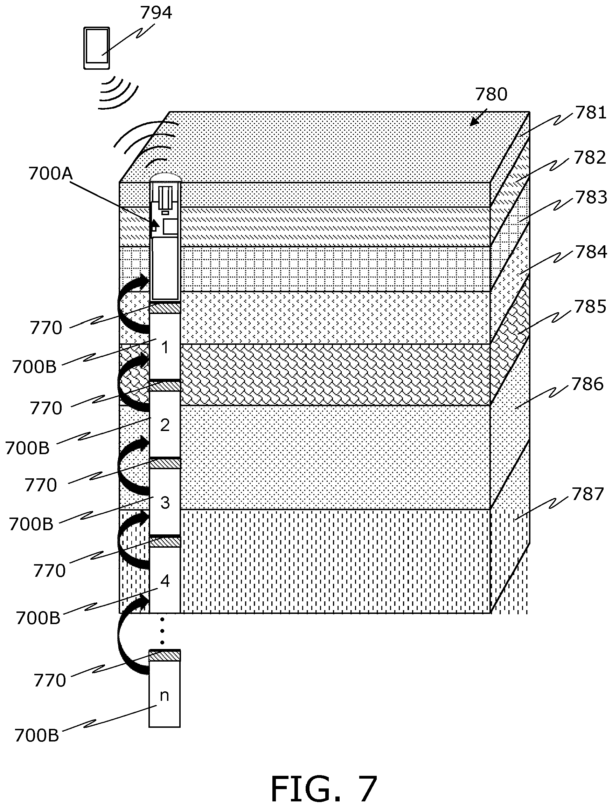

The summary above, as well as the following detailed description of illustrative embodiments, is better understood when read in conjunction with the appended drawings. For the purpose of illustrating the present disclosure, exemplary constructions of the embodiments of the disclosure are shown in the drawings, with references to the following diagrams wherein:
Figure FIG. 1A is a top view and figure FIG. 1B is a cross-sectional view of a ground monitoring device according to an embodiment of the present disclosure;
Figure FIG. 2A is a side view and figure FIG. 2B is a cross-sectional view of a ground monitoring device according to a second embodiment of the present disclosure;
Figure FIG. 3A is a cross-sectional view and figure FIG. 3B is a top view of a ground monitoring device according to a third embodiment of the present disclosure;
Figure FIG. 4 is a schematic illustration of a ground monitoring device according to an embodiment of the present disclosure;
Figure FIG. 5 is an illustrative view of ground monitoring devices according to the present disclosure installed into a road;
Figure FIG. 6 is an illustrative view of a ground monitoring device according to an embodiment of the present disclosure installed into a bridge;
Figure FIG. 7 is an illustrative view of a mesh network of ground monitoring devices according to an embodiment of the present disclosure installed into a road.

### DETAILED DESCRIPTION OF EMBODIMENTS

The following detailed description illustrates embodiments of the present disclosure and ways in which they can be implemented. Throughout the present disclosure the term "ground", if not specified, as used herein in general refers to the roads, pavements, bridges, viaducts, agricultural soils and fields, sports grounds and the like.

In an aspect, an embodiment of the present disclosure provides a ground monitoring device for measuring ground surface data comprising a hollow cylindrical housing having a first end and a second end, wherein the hollow cylindrical housing comprises a power unit in the first end, and an electronics module in the second end, wherein the electronics module comprises at least one conductive element comprising one or more first sensors, a radio transmitter, a power control real time clock, a computing element.

The ground surface data measured by the ground monitoring device according to the embodiments of the present disclosure can be used for road maintenance planning and quality analysis, mobility and transportation planning, road weather applications, road condition forecasting (e.g., what happens with the road surface in the future), severe weather warning services (e.g., when the weather gets extreme, monitoring flooding, overheating and freezing conditions etc), route planning and travel time prediction (e.g., how weather affects travel times), road construction (e.g., avoiding downtimes) , road safety monitoring (e.g., paved pathways, parking areas, stairs etc), traffic flow analysis.

Agricultural ground surface data measured by the ground monitoring device according to the embodiments of the present disclosure can be used for farming, greenhouses, horticulture, scientific research. The ground monitoring device can be further configured to monitor surface data of golf fields, soccer fields, and other sports fields.

The ground monitoring device is configurable to communicate data to other communication units like weather stations, other sensors and devices (e.g., servers, computing devices, vehicle's on-board computers, smartphones and other user devices capable to receive data) for transferring the collected raw measurement data for data processing and data analysis.

For measuring the data, the ground monitoring device is installed into the ground. Depending on the pre-determined measurement period (e.g., measuring the data in every 3, 5, 7, etc minutes) the lifetime of the ground monitoring device may be 3-5 years. Thus, the device is configurable to measure one or more ground parameters; to send the measured one or more ground parameters to one or more data communication units; and optionally configured to receive data from one or more data communication units.

The cylindrical housing of the ground monitoring device may be made of plastic, such as polyethylene terephthalate glycol (PETG). The plastic may be black or dark plastic. Such material of the housing enables the ground monitoring device to wear down together with the ground surface so that when the ground surface is wearing down it does not damage the components of the ground monitoring device and the ground monitoring device is still operable. This is especially advantageous when the ground monitoring device is installed into the road surface. The diameter of the housing may be up to 5-20 millimetres (mm). The diameter of the housing can be thus from 5, 6, 7, 8, 9, 10, 11, 12, 13, 14, 15, 16, 17, 18 or 19 mm up to 6, 7, 8, 9, 10, 11, 12, 13, 14, 15, 16, 17, 18, 19 or 20 mm. The height of the housing of the ground monitoring device may be 30-100 mm. The height of the housing of the ground monitoring device can be thus from 30, 35, 40, 45, 50, 55, 60, 65, 70, 75, 80, 85, 90 or 95 mm up to 35, 40, 45, 50, 55, 60, 65, 70, 75, 80, 85, 90, 95 mm. In an embodiment, the diameter may be 15 mm and length 60 mm, which is optimized for road surface data measurement. For monitoring agricultural data, the length may be 40-50 mm. The dimensions of the ground monitoring device allow the ground monitoring device to adapt to the deformations of the road surface.

When installed to tarmac or pavement, the black or dark plastic matches the visual and thermal properties of single gravel rock inside the asphalt. Matching the thermal properties of the road surface enables to improve the measurement accuracy.

The ground monitoring device is adapted to behave according to the road condition changes, e.g., temperature changes. The housing made of plastic enables to react to the changes in the road due to elastic properties of the plastic, e.g., polyethylene terephthalate glycol (PETG), which further improves impact resistance and durability of the ground monitoring device. These features improve the lifetime of the ground monitoring device.

Reducing the diameter of the sensor enables to match the ground monitoring device to rock sizes used in tarmac. This also enables the possibility to use simple tools (e.g., drill) for sensor installation that will minimize damages to the road and significantly reduce time needed for installation. The dimensions of the present wireless ground monitoring device enable to reduce the road damages and the time necessary for installation and thus improve the measurement accuracy and safety of the works.

The dimensions of the present are important because the ground monitoring device on the structures, e.g., viaducts) where construction and/or surface layers are thin. E.g., on bridges and viaducts, as it fits into the two upper layers (80-120 mm), namely into wear layer and intermediate layer and the installation of the ground monitoring device does not damage the protective layer. Due to the thin construction, bridges cool down faster than the road. Temperature and other conditions on the bridges differs from surrounding roads and often the first slipperiness occurs on the bridge, either due to the freezing of moisture or frost. Therefore, it is important that measurements can also be performed on bridges.

In the present ground monitoring device, the internal heat dissipation and sensor volume are significantly reduced. This enables to improve the measurement quality, reduce road damages and install the sensor on thin constructions.

The power unit of the ground monitoring device may be a battery, such as a 3,6V LiSOCL2 battery. The electronics module of the ground monitoring device is arranged to accommodate electrical components of the ground monitoring device to control the power consumption, and to control the data measurements and data transfer.

The components of the electronics module of the ground monitoring device may be covered with black or grey epoxy. The at least one conductive element comprising a hollow body having an open end and a closed end and comprising one or more first sensors. The at least one conductive element may be a single conductive element. In an embodiment, the conductive element is arranged to act as a heat conductor and conduct a temperature of the ground surface (e.g., road surface) to the temperature sensor. The one or more first sensors is selected from the group of sensors comprising a temperature sensor, a water content detection sensor, a salinity detection sensor, a conductivity sensor, a capacitive sensor. The conductive element is connected to electronics via a single soldering pad.

The radio transmitter of the ground monitoring device is configured to transmit the monitored data to one or more data communication units. The one or more data communication units may be in different embodiments one or more of weather stations, user applications and vehicles. The radio transmitter may be a long-range Bluetooth antenna. Alternatively, or additionally a capacitive touch controller may be used as a radio transmitter. To optimize the data transfer and reduce the power consumption a wireless over-the-air data transmission instead of the cable is used according to the embodiment. I.e., the transfer of the data from the ground monitoring device to the data communication unit (e.g., moving vehicle or an operator carrying a data communication unit) may not be relevant to the actual new location of moving data communication unit. Creating a network data layer using ground monitoring devices communication capabilities readable from the air, such as allowing a GSM or radio signal to be read, i.e., reaching the user in a minimum amount of time.

The power control real time clock (RTC) of the ground monitoring device may be ultra-low power (<50nA) Real-time clock and calendar with programmable wakeup time. The computing element of the ground monitoring device is e.g., a microcontroller unit (MCU), computer on a chip, Internet of things (IoT) module or the like. According to the embodiments of the present disclosure, the computing element is configured to control logic of the ground monitoring device, to manage the sensors, to perform analytics, to perform ground surface data measurements, to control Bluetooth.

According to the embodiments, the computing element is configured at least to schedule Real-time clock output signal on and off for controlling power on and off status of the computing element; charge the at least one sensor capacitor via a resistor with defined resistance; determine liquid content on top of the pavement; determine salt content on top of the pavement.

The RTC is configured to control power to the computing element and other electronics, which enables to reduce power consumption significantly. The low power RTC may be pre-programmed to be power control and activation trigger for computing element. This way only the extremely low power RTC is the power consumer for 99,5% of the time enabling long lasting solutions.

Power consumption is a critical parameter affecting lifetime, performance, shape and size of the ground monitoring device. Ultra-low power Real-time clock and calendar (RTC) consumes less power than computing element in "deep sleep" or low-power mode. This enables to configure the ground monitoring device so that the computing element is not directly connected to battery power. Initial power to the computing element is thus provided by a software loading tool during software loading and testing. The RTC output signal on and off schedule is configured by the computing element during computing element software loading to switch on the power to the computing element according to the predefined schedule.

In other words, initial powering of the computing element of the ground monitoring device is performed by programming and software loading tool, whereby the computing element is initiated to configure a control signal output schedule of the RTC and to switch the power to the computing element on and off according to the schedule. When the power to the computing element is switched on by the RTC, the computing element is configured to perform the ground surface data measurements, after the measurement cycle the RTC is configured to switch the power to the computing element off. When the power is switched off, the RTC is in low power mode and configured to count the schedule when to switch power to the computing element on next time. Using the RTC as power control thus enables to optimize power consumption of the ground monitoring device.

In an embodiment the computing element of the ground monitoring device is configured to communicate with one or more weather stations, wherein the ground monitoring device is configured to send the ground monitoring data to the one or more weather stations which transfer the monitored ground surface data along with other weather data to a server. In the server by using the received ground surface and weather data (e.g., dew point temperature), slip hazard, slipperiness and road condition prediction, route optimization and recommendations for road maintenance machines can be calculated.

In an embodiment the ground monitoring device is configured to communicate with one or more mobile ground surface data receiver comprising a processor. The mobile ground surface data receiver may be e.g., a vehicle or an operator carrying a data communication unit configured to communicate with the ground monitoring device. The ground monitoring device is configured to send the ground monitoring data to the processor of the mobile ground surface data receiver, wherein based on the received data the processor is configured to calculate e.g., local risk of slipperiness, correction of speed, acceleration, traction control, driver assistance, direction of vehicles.

In an embodiment the ground monitoring device is configured to communicate with one or more weather stations, wherein the ground monitoring device is configured to send the ground monitoring data to the one or more weather stations, wherein based on the received ground surface data the weather station is configured to perform analysis of soil moisture, temperature and soil type, analysis of irrigation needs, assessment of susceptibility to fertilizer and plant protection, assessment of suitable plant varieties, detailed planning of timed activities (e.g., when to cultivate, etc.), local weather forecast.

In an embodiment the ground monitoring device is configured to communicate with one or more mobile ground surface data receiver comprising a processor. The mobile ground surface data receiver may be e.g., tractor or some other agricultural machinery comprising a computing device. The ground monitoring device is configured to send the ground monitoring data to the computing device of the agricultural machinery, wherein based on the received ground surface data the weather station is configured to perform analysis of soil moisture, temperature and soil type, analysis of irrigation needs, assessment of fertility and plant protection susceptibility, assessment of suitable plant varieties, planning of timed and localized activities (e.g., when to cultivate, etc.), adjustment of driving (speed, acceleration, traction control, driver assistance, direction) of the machinery.

In an embodiment, the ground monitoring device comprises one or more second sensors. According to the embodiments, the one or more second sensor is selected from a group of sensors comprising a gyroscope sensor, a tilt sensor, a pressure sensor, an inductive sensor, a microwave sensor, a light sensor, a pH and EC measurements sensor, a proximity sensor, a gas sensor, an IR sensor, a PIR sensor, a smoke sensor, a CO2 sensor, a CO sensor and an ultrasonic sensor.

The second sensor of the ground monitoring device may be a tilt sensor or a gyroscope sensor. The second sensor enables to determine the position of the ground monitoring device when the ground monitoring device is installed into the ground. The determined position of the ground monitoring device is then used to activate and deactivate the ground monitoring device. While the ground monitoring device is placed horizontally or the ground monitoring device has sunk to the side, the power of the ground monitoring device is cut off and ground monitoring device is inactive. While the ground monitoring device is placed vertically, the ground monitoring device is activated. I.e., the tilt sensor or the gyroscope sensor enables do detect the position of the ground monitoring device and thus can be configured to be switched off or switched on based on the position. The ground monitoring device may, based on the detected position, further be configured to start data synchronisation with the communication unit or some other device operable as monitored ground surface data receiver.

The proximity sensor enables to measure the distance between the one or more ground monitoring devices and the distance between the ground monitoring device and a data communication unit (i.e., weather stations, vehicle's on-board computers, smartphones and other user devices).

In an embodiment, the at least one conductive element is placed on top of the ground monitoring device. In the embodiment, for measuring a road surface data, the conductive element is placed on top of the ground monitoring device for monitoring road temperature and water content. This enables to make the ground monitoring device smaller in diameter to match rock sizes used in tarmac or pavement. Such placement of the conductive element further enables efficient temperature and water content sensing of the road surface.

In another embodiment, the at least one conductive element is placed around the second end of the ground monitoring device. Such embodiment enables to monitor the ground data for agricultural purposes and measure temperature and humidity of the e.g., soil, grass. The conductive element may be thus implemented as a continuous ring.

Optionally, according to some embodiments of the present disclosure, the ground monitoring device comprises one or more stacking pins. In some embodiments two or more ground monitoring device are adapted to be vertically stackable for forming a mesh network of ground monitoring devices. Such mesh network of ground monitoring devices may comprise at least one ground monitoring device for monitoring the road surface data (i.e., a first ground monitoring device) and a set of ground monitoring devices comprising one or more ground monitoring devices (i.e., a second ground monitoring device) for monitoring data of one or more layers of the ground. The stacking pins enable to stack two or more ground monitoring devices on top of each other to form the mesh network of ground monitoring devices. Such a mesh network of ground monitoring devices enables to monitor ground data from different layers of the ground.

Different layers of the ground may be for example layers of the road such as asphalt surface or tarmac, asphalt base, asphalt binder, drainable base, sub-grade, embankment fill, natural soil. The two or more vertically stacked ground monitoring devices thus enable to measure temperature and humidity incrementally between surface and inside the ground (up and down to several meters). Such measurement enables to detect how deep is the freezing of the road, how deep the water is in the road.

In such embodiment the first ground monitoring device in the stack at the ground surface side comprises a conductive element placed to the second end on top of the ground monitoring device and at least one ground monitoring devices comprising conductive element placed on the side of the ground monitoring device is connected by one or more stacking pins to the first ground monitoring device for measuring temperature and humidity sensing of the road body internal level or different layers of the soil.

Optionally, the ground monitoring device comprises a cylindrical flexible Printed Circuit Board. In the embodiment the cylindrical flexible Printed Circuit Board is placed at the outer side of the ground monitoring device as antenna to extend the wireless connectivity range from inside the road. Due to the materials in the road, e.g., asphalt, concrete, gravel, sand etc., the radio (e.g., Bluetooth) signal is significantly blocked. Wireless data range from inside the road and tarmac is severely affected due to the physical and chemical parameters of the road. This results in significant loss of radio transmission range and reduces battery lifetime. To mitigate this issue, surround the outer range of the ground monitoring device with a cylindrical flexible Printed Circuit Board (PCB) that is connected to ground. This enables to extend wireless connectivity range from inside the ground when sensors are applied to different soils or tarmacs etc. It also provides physical protection and protection for the electrical field and helps to stabilize the ground monitoring device.

According to the embodiments, the ground monitoring device may comprise a power converter element. The power converter element of the ground monitoring device is adapted to harvest road temperature for powering the ground monitoring device. This enables to avoid using extensive batteries or charging solutions, save the power consumption and increase the life time of the ground monitoring device. The power converter element may be implemented by using heat collected into the road (solar radiation, ground heat) and to convert it into electrical power for the sensor of the ground monitoring device.

Optionally, the ground monitoring device may further comprise one or more data receivers. The data receiver enables to make the ground monitoring device autonomous by connecting the ground monitoring device to one or more data communication units. The one or more data communication unit may be a weather station, a gateway, a vehicle, a user device (e.g., laptop, tablet, smartphone or other portable device), a variable message sign or a server. This is achieved by using edge computing and Bluetooth, mobile or satellite connectivity enable autonomous setup with different simultaneous data communication units (weather station, smartphone, vehicle etc). Such embodiment does not need additional means, data conversion or logic unit to enable dada communications and makes the ground monitoring more efficient. The embodiment comprising one or more data receivers further enables to keep the data updated and in line with the actual conditions.

According to the embodiments, the radio transmitter may be configured to transmit data periodically. Configuring the radio transmitter of the ground monitoring device to transmit the data with predefined intervals is enabled by turning on the data transmission periodically. This enables to keep the data transmission not constantly active and thus to reduce the power consumption. In an embodiment, ground monitoring devices according to the present disclosure are installed to the ground and communicatively connected to a data communication unit, i.e., a data receiver, which may be e.g., a weather station. The ground monitoring devices are arranged to monitor the ground surface data periodically, i.e., to take e.g., temperature and humidity measurements, send the measurements to the server over the network etc. Between taking the measurements, the ground monitoring device is configured to be itched into sleep mode. The weather station is configured to scan availability of the ground monitoring devices at its range. In case the presence of ground a monitoring device is detected, the weather station and the one or more ground monitoring devices are automatically synchronized.

According to the embodiment, the predefined data transmission interval from the ground monitoring device to the weather station, may be e.g., 5 minutes. Initial scanning duration by weather station to catch the sequence of ground monitoring device data can be e.g., 6 minutes. Next data scanning window after the previous successful data receiving timestamp may be 299 700 milliseconds to 300 300 milliseconds. In case the weather station is not able to receive data for e.g., 3 consecutive occasions, the weather station initializes 6 minutes scanning window again. The ground monitoring device is configured to send the last e.g., 4 hours of data with every package, therefore all the recent small gaps in data will be filled with the next successful transfer.

As the data communication unit (e.g., the weather station) also operates on a limited power source, detecting the advertising data packets from the ground monitoring side periodically enables also to reduce the power consumption of the data receiver.

A further reduction in power consumption may be achieved if in case than two or more ground monitoring devices on site connected to the data communication unit are configured to be active and in sleep mode at different periods.

In an embodiment, the at least one conductive element comprises a hollow body having an open end and a closed end and wherein the at least one conductive element is configured at least to transfer ground surface temperature to a temperature sensor, and to measure voltage.

The closed end of the conductive element is placed at the surface side when the ground monitoring device is installed into the ground, road or soil. The hollow body having the open end and the closed end of the conductive element enables to react according to the changes in the environment and measure the temperature in a range of -40 degrees up to +80 degrees by Celsius. The form factor of the conductive element and dark or grey color of the housing of the ground monitoring device is adapted to fit to the soil and the color of the road surface to ensure even warming in the sun and the lowest possible heat capacity to respond to rapid changes in the soil or the surrounding environment.

The hollow body of the conductive element may be made of brass. The hollow body of the conductive element enables better transition of highspeed electrical signals and less heat capacity for rapid response of environment changes.

The form factor, size and color of the heat conductive element thus enables the fastest possible heat transfer from the ground or road surface to the sensor measuring element.

In an embodiment the conductive element can be placed at the surface of the second end of the housing. In another embodiment the conductive element can be placed up to 3-5 millimeters lower from the surface of the second end of the housing. Placing the conductive element lower from the surface of the second end of the housing ensures that the ground monitoring device is still operable when the housing wears down due to the ground or road surface wear.

The ground monitoring device thus enables to detect liquids content, to detect thickness of the liquid layer on the ground and salinity of the liquids and to transfer ground or road surface temperature to the temperature sensor of the ground monitoring device. The ground monitoring device enables to determine the thickness of the water layer on the ground up to 50 millimeters.

Typical liquids detection requires several contact points and thus multiple elements for the measurements and therefore has higher demand for power and physical volume consumption. According to the embodiments of the present disclosure, the liquids content is detected by the conductive element, which is connected to the electronics inside the ground monitoring device. The conductive element of the ground monitoring device is adapted to act as a capacitor and is connected to the computing element for analog sensing signal and to the resistor, wherein the resistor is being connected to the computing element for charge generation signal.

The conductive element is adapted to form with the ground surface a capacitor. The conductive element further comprises an output pin of computing element for charging the capacitor via a resistor with defined resistance and one analog input pin connected to the electrode at the same point as the GPIO (without the resistor). Such tuned capacitive Resistor-Capacitor charge timing enables to detect liquids content with low power consumption and therefore increases the lifetime of the ground monitoring device.

The computing element is configured to obtain from the conductive element a relative reading of a Volumetric Water Content (VWC). The VWC may be in a range of 0-8000. The Volumetric Water Content indicates the soil moisture content, i.e., how much of the soil volume is water.

This is used to measure the voltage over the capacitor. Voltage change characteristics are analyzed by computing element to determine moisture level on top of the ground and to determine surface salt content (salt inclusion level) on top of the ground.

In another aspect, an embodiment of the present disclosure provides a computer program for measuring ground surface data comprising instructions which when the computer program is executed by a ground monitoring device comprising a computing element, cause the ground monitoring device to measure ground surface data.

The computer program of the ground monitoring device for measuring ground surface data comprises one or more software components which enable pre-programming of the computing element of the ground monitoring device to perform the ground data measurements, control and manage the work of the electronic components of the ground monitoring device, to communicate with external devices, to transmit the measured ground data to the external devices, to control the Real-time clock and calendar with pre-programmable wakeup time, to carry out the calculations and interpretations of measured ground surface data.

The computing element comprising the computer program of the ground monitoring device is further pre-programmed to analyse voltage change characteristics to determine liquids content and salinity.

The computer program further enables to configure the computing element of the ground monitoring device to schedule Real-time clock output signal on and off for controlling power on and off status of the computing element; to charge the at least one sensor capacitor via a resistor with defined resistance; to determine moisture level on top of the ground; to determine salt inclusion level on top of the ground.

### DETAILED DESCRIPTION OF THE DRAWINGS

Referring to figure FIG. 1A there is shown a top view and figure FIG. 1B is a cross-sectional view of a ground monitoring device **100** according to an embodiment of the present disclosure. The ground monitoring device **100** for measuring ground surface data comprising a hollow cylindrical housing **110** having a first end **112** and a second end **114.** The hollow cylindrical housing comprises a power unit **120** in the first end **112** and an electronics module **130** in the second end **114.** The electronics module **130** comprises a conductive element **131** comprising one or more first sensors, a radio transmitter **132,** a power control real time clock **134,** a computing element **135,** and a tilt sensor as a second sensor **133.** In the embodiment the conductive element **131** is placed on top **150** of the ground monitoring device at the ground surface side.

Referring to figure FIG. 2A there is shown a side view and figure FIG. 2B is a cross-sectional view of a ground monitoring device **200** according to a second embodiment of the present disclosure. In the embodiment, the ground monitoring device **200** for measuring ground surface data comprising a hollow cylindrical housing **210** having a first end **212** and a second end **214.** The hollow cylindrical housing comprises a power unit **220** in the first end **212** and an electronics module **230** in the second end **214.** The electronics module **230** comprises a conductive element **231** comprising one or more first sensors, a radio transmitter **232,** a power control real time clock **234,** a computing element **235.** In the embodiment, the conductive element **231** is placed around the second end **214** of the ground monitoring device.

Referring to figure FIG. 3A there is shown a cross-sectional view and figure FIG. 3B is a top view of a ground monitoring device **300** according an embodiment of the present disclosure. The ground monitoring device **300** for measuring ground surface data comprising a hollow cylindrical housing **310** having a first end **312** and a second end **314,** wherein the hollow cylindrical housing comprises a power unit **320** in the first end **312,** and an electronics module **330** in the second end **314.** The electronics module **330** comprises a conductive element **331** comprising one or more first sensors, a radio transmitter **332,** a power control real time clock **334,** and a computing element **335.** In the embodiment, the ground monitoring device **300** further comprises a cylindrical flexible Printed Circuit Board **350** operating as a shielding.

Referring to figure FIG. 4 there is shown a schematic illustration of a ground monitoring device **400** according to an embodiment of the present disclosure. According to the embodiment ground surface moisture detection and ground surface salt content detection using capacitive Resistor-Capacitor charge timing is illustrated. The ground monitoring device 400 according to the embodiments of the present disclosure comprises a conductive element **441** and a computing element **435.** The conductive element **441** comprises a hollow body having an open end **442** and a closed end **443.** The conductive element **441** is adapted to form with the ground surface **446** a capacitor **C.** The conductive element **441** is connected to the computing element **435** for and to the resistor R **445,** wherein the resistor **445** is connected to the computing element **435.**

Referring to figure FIG. 5 there is shown an illustrative view of ground monitoring devices according to the present disclosure installed into a road. The ground monitoring devices **500** are installed to the road **591** at a first location **500a,** at a second location **500b,** at a third location **500c,** at a fourth location **500d.** The ground monitoring devices **500** at locations **500a, 500b, 500c, 500d** are configured to measure the road **591** surface data at corresponding locations and to communicate for the measured road surface data transfer with data communication units such as vehicle **592,** weather station **593** and user device **594.**

Referring to figure FIG. 6 there is shown an illustrative view of a ground monitoring device **600** according to an embodiment of the present disclosure installed into a bridge **691** comprising a wear layer about 5cm **691a,** intermediate layer about 7cm **691b,** protective layer about 3.5cm **691c,** hydro insulation layer about 1cm **691d,** a concrete layer about 50-60cm **691d.** As shown in the figure, the ground monitoring device according to the present disclosure can be installed on bridges as it fits into the two upper layers, namely into wear layer and intermediate layer and does not damage the protective layer.

Referring to figure FIG. 7 there is shown an illustrative view of a mesh network of ground monitoring devices **700** according to an embodiment of the present disclosure installed into a road **780** comprising first asphalt polymer layer **781,** second asphalt polymer layer **782,** asphalt binder **783,** asphalt base **784,** drainable base **785,** sub-grade **786,** natural soil **787.** In such embodiment, the ground monitoring device comprises one or more stacking pins **770.**

The mesh network of ground monitoring devices is formed by connecting a first ground monitoring device **770A** comprising stacking pins **770** vertically with a set of second ground monitoring devices comprsing multiple second ground monitoring devices **770B.** Each of the second ground monitoring devices of the set of second ground monitoring devices comprise stacking pins **770.**

In the embodiment the first ground monitoring device **770A** of the mesh network of ground monitoring devices is installed into the upper layers of the road **780** (e.g., first asphalt polymer layer **781,** second asphalt polymer layer **782,** asphalt binder **783,** asphalt base **784)** and adapted to measure temperature on top of the road **780** surface. The vertically stacked set of second ground monitoring devices **770B** are adapted to measure temperature and humidity of the lower layers of the road (e.g., asphalt base **784,** drainable base **785,** sub-grade **786,** natural soil **787).** The second ground monitoring devices **770B** in the set of second ground monitoring devices are configured so that the measurement data of the layers is transferred from the lowest second ground monitoring device n in the set of second ground monitoring devices to the upper second ground monitoring devices up to the first ground monitoring device **770A,** which is configured to transfer the measured ground data collected from each ground monitoring device in the vertical stack to the mobile user device **794.**

## Claims

1. A ground monitoring device (100, 200, 300, 400, 500, 600, 700A, 700B) for measuring ground surface data comprising
a hollow cylindrical housing (110, 210, 310) having a first end (112, 212, 312) and a second end (114, 214, 314), wherein the hollow cylindrical housing comprises
a power unit (120, 220, 320) in the first end (112, 212, 312), and an electronics module (130, 230, 330) in the second end (114, 214, 314), wherein the electronics module (130, 230, 330) comprises
at least one conductive element (131, 231, 331, 441) comprising one or more first sensors,
a radio transmitter (132, 232, 332),
a power control real time clock (134, 234, 334),
a computing element (135, 235, 335, 435).

2. The ground monitoring device according to claim 1, wherein the ground monitoring device further comprises one or more second sensors (133).

3. The ground monitoring device according to claim 1 or 2, wherein the at least one conductive element (131) is placed on top (150) of the ground monitoring device.

4. The ground monitoring device according to claim 1 or 2, wherein the at least one conductive element (231) is placed around the second end (214) of the ground monitoring device.

5. The ground monitoring device according to claim 3 or 4, wherein the ground monitoring device comprises one or more stacking pins (770).

6. The ground monitoring device according to any of preceding claims, wherein the ground monitoring device comprises a cylindrical flexible Printed Circuit Board (350).

7. The ground monitoring device according to any of preceding claims, wherein the ground monitoring device comprises a power converter element.

8. The ground monitoring device according to any of preceding claims, wherein the one or more second sensor is selected from a group of sensors comprising a gyroscope sensor, a tilt sensor, a pressure sensor, an inductive sensor, a microwave sensor, a light sensor, a pH and EC measurements sensor, a proximity sensor, a gas sensor, an IR sensor, a PIR sensor, a smoke sensor, a C02 sensor, a CO sensor and an ultrasonic sensor.

9. The ground monitoring device according to any of preceding claims, wherein the ground monitoring device further comprises one or more data receivers.

10. The ground monitoring device according to any of preceding claims, wherein the radio transmitter (132, 232, 332) is configured to transmit data periodically.

11. The ground monitoring device according to any of preceding claims, wherein the at least one conductive element (441) comprising a hollow body having an open end (442) and a closed end (443) and wherein the at least one conductive element is configured at least to transfer ground surface temperature to a temperature sensor, and to measure voltage.

12. The ground monitoring device according to any of preceding claims, wherein the computing element is configured at least to schedule Real-time clock output signal on and off for controlling power on and off status of the computing element;
charge the at least one sensor capacitor via a resistor with defined resistance;
determine moisture level on top of the ground;
determine salt inclusion level on top of the ground.

13. A computer program for measuring ground surface data comprising instructions which when the computer program is executed by a ground monitoring device (100, 200, 300, 400, 500, 600, 700A, 700B) comprising a computing element (135, 235, 335, 435), cause the ground monitoring device to measure ground surface data.
